# EUROPEAN PATENT SPECIFICATION

(11) **EP 4 444 699 B1**
(45) Date of publication and mention of the grant of the patent: **21.10.2026**
(21) Application number: 22835607.7
(22) Date of filing: 09.12.2022
(51) Int. Cl.: C07D 213/61, A61K 31/44, A61P 3/10, A61K 45/06

(54) **CRYSTALLINE FORM OF (R)-2-(TERT-BUTYLAMINO)-1-(5-FLUOROPYRIDIN-3-YL)-ETHAN-1-OL HEMI-TARTRATE SALT FOR THE TREATMENT OF HYPERGLYCEMIA AND DIABETES 2**
KRISTALLINE FORM DES (R)-2-(TERT-BUTYLAMINO)-1-(5-FLUOROPYRIDIN-3-YL)-ETHAN-1-OL HEMI-TARTRAT SALZES ZUR BEHANDLUNG VON HYPERGLYKÄMIE UND DIABETES 2
FORME CRYSTALLINE DU SÉL HÉMI-TARTRATE DE (R)-2-(TERT-BUTYLAMINO)-1-(5-FLUOROPYRIDIN-3-YL)-ÉTHAN-1-OL POUR LE TRAITEMENT DE HYPERGLYCÉMIE ET DIABÈTE 2

(30) Priority: 09.12.2021 GB 202117828
(43) Date of publication of application: 16.10.2024
(60) Divisional application: 26204124.7
(73) Proprietor: Atrogi AB, 171 65 Solna (SE)
(72) Inventor: PELCMAN, Benjamin, SE113 52 Stockholm (SE)
(74) Representative: Potter Clarkson
(86) International application number: PCT/EP2022/085133
(87) International publication number: WO 2023/105035

(56) References cited:
- WO-A1-2019/053427

## Description

### Field of the Invention

The present invention relates to novel salts and compositions, and said salts and compositions for use in methods of treatment of hyperglycaemia and disorders characterised by hyperglycaemia, such as type 2 diabetes. In particular, the invention relates to novel salts, compositions and methods for the treatment of conditions such as type 2 diabetes through activation of the β₂-adrenergic receptor. Importantly, such salts are thought to have a beneficial side-effect profile as they do not exert their effect through significant cAMP release.

### Background of the Invention

The listing or discussion of an apparently prior-published document in this specification should not necessarily be taken as an acknowledgement that the document is part of the state of the art or is common general knowledge.

Hyperglycaemia, or high blood sugar is a condition in which an excessive amount of glucose circulates in the blood plasma. If not treated, hyperglycaemia can be a serious problem, potentially developing into life-threatening conditions such as ketoacidosis. For example, chronic hyperglycemia may cause injury to the heart, and is strongly associated with heart attacks and death in subjects with no coronary heart disease or history of heart failure. There are various causes of hyperglycaemia, including diabetes and severe insulin resistance.

Severe insulin resistance (SIR) is a condition wherein the patient experiences very low levels of (or, in extreme cases, no significant) response to insulin. There are several syndromes characterized by SIR, including Rabson-Mendenhall syndrome, Donohue's syndrome (leprechaunism), Type A and Type B syndromes of insulin resistance, the HAIR-AN (hyperandrogenism, insulin resistance, and acanthosis nigricans) syndrome, pseudoacromegaly, and lipodystrophy. The majority of these conditions have genetic causes, such as mutations in the insulin receptor gene. The prevalence for Donohue's syndrome, Rabson-Mendenhall syndrome and Type A syndrome of insulin resistance, has been reported to vary from about 50 reported cases to 1 in 100,000. However, since some diseases are severe and extremely rare, it is likely that many patients do not get diagnosed before they die, particularly in less developed areas of the world. Thus, the exact number of patients with these syndromes is difficult to assess.

The current standard for hyperglycaemia treatment in patients having SIR is a controlled diet, supplemented with drugs affecting insulin receptor sensitivity, such as metformin, or insulin supplement. However, particularly for disorders caused by mutations in the insulin receptor gene, this treatment is not sufficiently effective and ultimately proves unsuccessful.

Diabetes comprises two distinct diseases, type 1 (or insulin-dependent diabetes) and type 2 (insulin-independent diabetes), both of which involve the malfunction of glucose homeostasis. Type 2 diabetes affects more than 400 million people in the world and the number is rising rapidly. Complications of type 2 diabetes include severe cardiovascular problems, kidney failure, peripheral neuropathy, blindness and, in the later stages of the disease, even loss of limbs and, ultimately death. Type 2 diabetes is characterized by insulin resistance in skeletal muscle and adipose tissue, and there is presently no definitive cure. Most treatments used today are focused on remedying dysfunctional insulin signalling or inhibiting glucose output from the liver but many of those treatments have several drawbacks and side effects. There is thus a great interest in identifying novel insulin-independent ways to treat type 2 diabetes.

In type 2 diabetes, the insulin-signalling pathway is blunted in peripheral tissues such as adipose tissue and skeletal muscle. Methods for treating type 2 diabetes typically include lifestyle changes, as well as insulin injections or oral medications to regulate glucose homeostasis. People with type 2 diabetes in the later stages of the disease develop 'beta-cell failure' i.e. the inability of the pancreas to release insulin in response to high blood glucose levels. In the later stages of the disease patients often require insulin injections in combination with oral medications to manage their diabetes. Further, most common drugs have side effects including downregulation or desensitization of the insulin pathway and/or the promotion of lipid incorporation in adipose tissue, liver and skeletal muscle. There is thus a great interest in identifying novel ways to treat metabolic diseases including type 2 diabetes that do not include these side effects.

Following a meal, increased blood glucose levels stimulate insulin release from the pancreas. Insulin mediates normalization of the blood glucose levels. Important effects of insulin on glucose metabolism include facilitation of glucose uptake into skeletal muscle and adipocytes, and an increase of glycogen storage in the liver. Skeletal muscle and adipocytes are responsible for insulin-mediated glucose uptake and utilization in the fed state, making them very important sites for glucose metabolism.

The signalling pathway downstream from the insulin receptor has been difficult to understand in detail. In brief, control of glucose uptake by insulin involves activation of the insulin receptor (IR), the insulin receptor substrate (IRS), the phosphoinositide 3-kinase (PI3K) and thus stimulation of phosphatidylinositol (3,4,5)-triphosphate (PIP3), the mammalian target of rapamycin (also called the mechanistic target of rapamycin, mTOR), Akt/PKB (Akt) and TBC1D4 (AS160), leading to translocation of the glucose transporter 4 (GLUT4) to the plasma membrane. Akt activation is considered necessary for GLUT4 translocation.

It should be noted that skeletal muscles constitute a major part of the body weight of mammals and have a vital role in the regulation of systemic glucose metabolism, being responsible for up to 85% of whole-body glucose disposal. Glucose uptake in skeletal muscles is regulated by several intra- and extracellular signals. Insulin is the most well studied mediator but others also exist. For example, AMP activated kinase (AMPK) functions as an energy sensor in the cell, which can increase glucose uptake and fatty acid oxidation. Due to the great influence skeletal muscles have on glucose homeostasis it is plausible that additional mechanisms exist. In the light of the increased prevalence of type 2 diabetes, it is of great interest to find and characterize novel insulin-independent mechanisms to increase glucose uptake in muscle cells.

Blood glucose levels may be regulated by both insulin and catecholamines, but they are released in the body in response to different stimuli. Whereas insulin is released in response to the rise in blood sugar levels (e.g. after a meal), epinephrine and norepinephrine are released in response to various internal and external stimuli, such as exercise, emotions and stress, and also for maintaining tissue homeostasis. Insulin is an anabolic hormone that stimulates many processes involved in growth including glucose uptake, glycogen and triglyceride formation, whereas catecholamines are mainly catabolic.

Although insulin and catecholamines normally have opposing effects, it has been shown that they have similar actions on glucose uptake in skeletal muscle (Nevzorova et al., Br. J. Pharmacol, 137, 9, (2002)). In particular, it has been reported that catecholamines stimulate glucose uptake via adrenergic receptors (Nevzorova et al., Br. J. Pharmacol, 147, 446, (2006); Hutchinson, Bengtsson Endocrinology 146, 901, (2005)) to supply muscle cells with an energy-rich substrate. Thus it is likely that in mammals, including humans, the adrenergic and the insulin systems can work independently to regulate the energy needs of skeletal muscle in different situations. Since insulin also stimulates many anabolic processes, including some that promote undesired effects such as stimulation of lipid incorporation into tissues, leading to e.g. obesity, it would be beneficial to be able to stimulate glucose uptake by other means; for example, by stimulation of the adrenergic receptors (ARs).

All ARs are G protein-coupled receptors (GPCRs) located in the cell membrane and characterized by an extracellular N-terminus, followed by seven transmembrane α-helices (TM-1 to TM-7) connected by three intracellular (IL-1 to IL-3) and three extracellular loops (EL-1 to EL-3), and finally an intracellular C-terminus. There are three different classes of ARs, with distinct expression patterns and pharmacological profiles: α₁-, α₂- and β-ARs. The α₁-ARs comprise the α_{1A}, α_{1B} and α_{1D} subtypes while α₂-ARs are divided into α_{2A}, α_{2B} and α_{2C}. The β-ARs are also divided into the subtypes β₁, β₂, and β₃, of which β₂-AR is the major isoform in skeletal muscle cells. ARs are G protein coupled receptors (GPCRs) that signal through classical secondary messengers such as cyclic adenosine monophosphate (cAMP) and phospholipase C (PLC).

Many effects occurring downstream of ARs in skeletal muscles have been attributed to classical secondary messenger signalling, such as increase in cAMP levels, PLC activity and calcium levels. Stimulation involving the classical secondary messengers has many effects in different tissues. For example, it increases heart rate, blood flow, airflow in lungs and release of glucose from the liver, which all can be detrimental or be considered unwanted side effects if stimulation of ARs should be considered as a type 2 diabetes treatment. Adverse effects of classical AR agonists are, for example, tachycardia, palpitation, tremor, sweats, agitation and increased glucose levels in the blood (glucose output from the liver). It would thus be beneficial to be able to activate ARs without activating these classical secondary messengers, such as cAMP, to increase glucose uptake in peripheral tissues without stimulating the unwanted side effects.

Glucose uptake is mainly stimulated via facilitative glucose transporters (GLUT) that mediate glucose uptake into most cells. GLUTs are transporter proteins that mediate transport of glucose and/or fructose over the plasma membrane down the concentration gradient. There are fourteen known members of the GLUT family, named GLUT1-14, divided into three classes (Class I, Class II and Class III) dependent on their substrate specificity and tissue expression. GLUT1 and GLUT4 are the most intensively studied isoforms and, together with GLUT2 and GLUT3, belong to Class I which mainly transports glucose (in contrast to Class II that also transports fructose). GLUT1 is ubiquitously expressed and is responsible for basal glucose transport. GLUT4 is only expressed in peripheral tissues such as skeletal muscle, cardiac muscle and adipose tissues. GLUT4 has also been reported to be expressed in, for example, the brain, kidney, and liver. GLUT4 is the major isoform involved in insulin stimulated glucose uptake. The mechanism whereby insulin signalling increases glucose uptake is mainly via GLUT4 translocation from intracellular storage to the plasma membrane. It is known that GLUT4 translocation is induced by stimulation of the β₂-adrenergic receptor.

Thus, a possible treatment of a condition involving dysregulation of glucose homeostasis or glucose uptake in a mammal, such as type 2 diabetes, would involve the activation of the β₂-adrenergic receptor leading to GLUT4 translocation to the plasma membrane and promotion of glucose uptake into skeletal muscle leading to normalization of whole body glucose homeostasis. In addition, it would be advantageous if the treatment does not involve signalling through cAMP as this would lead to a favourable side-effect profile.

(R)-2-(tert-butylamino)-1-(5-fluoropyridin-3-yl)ethan-1-ol is disclosed in WO 2019/053427 and has been shown to act as an agonist at the β₂-adrenergic receptor, increasing glucose uptake in skeletal muscle but without leading to a significant release of cyclic adenosine monophosphate (cAMP).

There remains a need to improve the properties of active ingredients, such as (*R*)-2-(*tert-*butylamino)-1-(5-fluoropyridin-3-yl)ethan-1-ol, so as to improve their ease of use, particularly when used in the preparation of medicaments. In particular, there remains a need to provide active ingredients in a form having, inter alia, improved ease of formulation, improved stability, improved bioavailability, improved taste, improved ability to be scaled up, and/or lower hygroscopicity. There also remains a need to be able to obtain active ingredients as crystalline solids in a high yield and with high purity (in some circumstances including a high enantiomeric excess).

The listing or discussion of an apparently prior-published document in this specification should not necessarily be taken as an acknowledgement that the document is part of the state of the art or is common general knowledge.

### Description of the Invention

We have now surprisingly found that (R)-2-(tert-butylamino)-1-(5-fluoropyridin-3-yl)-ethan-1-ol (i.e. the compound of formula I as described below) may be prepared in a scalable manner in high yield as hemi-tartrate salts having high purity and high enantiomeric excess. In contrast, a number of other salt forms of this compound have either been not susceptible to crystallisation, obtainable in only low yields and/or low purities, found to have high hygroscopicity, unsuitable for use as pharmaceuticals (e.g. due to concerns regarding toxicity), and/or have been found to be unsuitable for scale-up production due to filtration problems.

It has been found that (R)-2-(tert-butylamino)-1-(5-fluoropyridin-3-yl)ethan-1-ol (i.e. the compound of formula I) acts as an agonist of the β₂-adrenergic receptor, increasing glucose uptake in skeletal muscle.

In addition, it has been found that this effect is not mediated through significant cAMP release, such that many of the commonly described side effects seen with traditional β₂-adrenergic agonists (e.g. tachycardia, palpitation, tremor, sweats, agitation, and the like) can be reduced.

The use of such salt forms of the compound as described herein in medicine represents a promising strategy for the treatment of conditions characterized by high blood sugar levels (i.e. hyperglycaemia), such as type 2 diabetes.

### Compounds of the invention

In a first aspect of the invention, there is provided a hemi-tartrate salt of a compound of formula I: 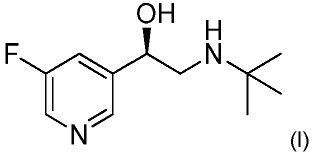 which compound of formula I may be referred to herein as "the compound of the invention" and which hemi-tartrate salts may be referred to herein as "salts of the invention".

For the avoidance of doubt, the skilled person will understand that references herein to salts of particular aspects of the invention (such as the first aspect of the invention, e.g. hemi-tartrate salts of a compound of formula I) will include references to all embodiments and particular features thereof, which embodiments and particular features may be taken in combination to form further embodiments.

For the avoidance of doubt, the compound of formula I is otherwise known by the IUPAC name (*R*)-2-(*tert*-butylamino)-1-(5-fluoropyridin-3-yl)ethan-1-ol.

Tartaric acid is otherwise known as 2,3-dihydroxybutanedioic acid or 2,3-dihydroxy-succinic acid, whose structure may be represented graphically as:

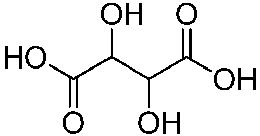

The skilled person will understand that tartaric acid may exist as three stereoisomers due to the two asymmetric carbon centres existing in the molecule.

(R,R)-tartaric acid (also known as dextrotartaric acid, (2R,3R)-tartaric acid, L-(+)-tartaric acid and L-tartaric acid) is one of a pair of enantiomers of tartaric acid, and has the structure which may be represented graphically as:

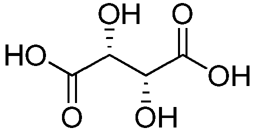

(S,S)-tartaric acid (also known as levotartaric acid, (2S, 3S)-tartaric acid, D-(-)-tartaric acid and D-tartaric acid) is the other of the pair of enantiomers of tartaric acid, and has the structure which may be represented graphically as:

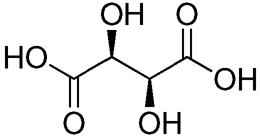

(2R,3S)-tartaric acid (also known as mesotartaric acid) is a stereoisomer of tartaric acid, and has the structure which may be represented graphically as:

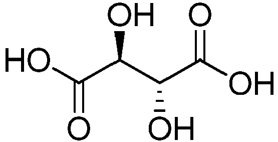

For the avoidance of doubt, all stereoisomers and mixtures thereof of tartaric acid (and, therefore, of tartrate) are included within the scope of the invention.

The term "tartrate" refers to the anion of tartaric acid. It will therefore be understood that the term "tartrate salt of a compound of formula I" refers to a chemical compound consisting of an assembly of anions of tartaric acid and associated cations of the compound of formula I.

Tartaric acid may exist as a monoanion (also known as the hydrogen tartrate anion or semi-tartrate anion) or as a dianion (also known as the tartrate dianion). Accordingly, as used herein, the term "tartrate" will be taken to include the tartrate monoanion, the tartrate dianion, and/or the mixtures thereof necessary to provide the appropriate charge balance within the salt.

As used herein, the term "hemi" in "hemi-tartrate salt of a compound of formula I" means that the stoichiometry between the compound of formula I and tartrate in the salt is 1:0.5 (i.e. equivalent to 2:1). Accordingly, the structure of the hemi-tartrate salt of the compound of formula I may, for example, be represented graphically as:

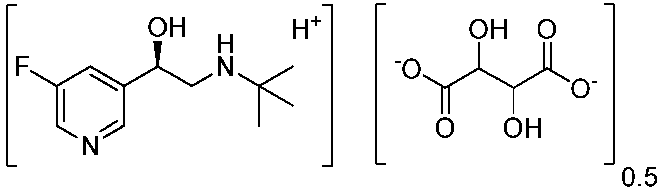

For the avoidance of doubt, salts of the first aspect of the invention are solid under ambient conditions, and thus the scope of the invention includes all amorphous, crystalline and part crystalline forms thereof.

Unless indicated otherwise, all technical and scientific terms used herein have the same meaning as commonly understood by one of ordinary skill in the art to which this invention pertains.

All embodiments of the invention and particular features mentioned herein may be taken in isolation or in combination with any other embodiments and/or particular features mentioned herein (hence describing more particular embodiments and particular features as disclosed herein) without departing from the disclosure of the invention.

In certain embodiments of the first aspect of the invention, the tartrate (i.e. the tartrate counterion present in the compound of the invention) comprises (2R, 3R)-tartrate.

In more particular embodiments, the tartrate salt consists essentially of (2R, 3R)-tartrate. By "consists essentially" we mean that the tartrate salt comprises at least 90% of the relevant form, such as at least 95% or at least 98%, e.g. at least 99%, such as at least 99.9%. Alternatively, the relevant stereochemical configuration (i.e. of the tartaric acid) may be referred to as being present in an enantiomeric excess (e.e.) and/or diastereomeric excess (d.e.), as appropriate, of at least 90% (such as at least 95%, at least 98% or, particularly, at least 99% or at least 99.5%; for example at least 99.9%).

In particular embodiments of the first aspect of the invention, the tartrate component of the salts of the invention is (2R, 3R)-tartrate.

The skilled person will understand that references to a specific steroisomer of a compound of formula I (i.e. where the carbon substituted by the essential -OH group is in the (R) configuration) may in specific emboidments refer to the specific stereoisomer present in the substantial absence of the corresponding opposite stereoisomer (i.e. where the carbon substituted by the essential -OH group is in the (S) configuration).

As used herein, references to the substantial absence of the corresponding opposite stereoisomer will refer to the desired stereoisomer being present at a purity of at least 80% (e.g. at least 90%, such as at least 95%) relative to the opposite stereoisomer. Alternatively, in such instances, compounds may be indicated to be present in the substantial absence of the compound in the other configuration (i.e. the (S) configuration), which may indicate that the compound in the relevant configuration is present in an enantiomeric excess (e.e.) of at least 90% (such as at least 95%, at least 98% or, particularly, at least 99% or at least 99.5%; for example at least 99.8%).

For the avoidance of doubt, references to the enantiomeric excess (e.e.) of the salts of the invention will refer to enantiomeric excess (e.e.) of the compound of formula I component the salts of the invention.

In certain embodiments, the relevant salt form (i.e. the salt form as claimed, being a combination of the compound of formula I and the tartaric acid) has a purity of greater than about 80% or 90%, preferably greater than about 95% or greater than about 98%, more particularly greater than about 99% (which values may if necessary be expresssed as ranges having as an upper point the maximum value achievable in the circumstances, which may be 100% or a value close to 100%, such as 99.5% or 99.9%, or particularly 99.99%).

In certain embodiments, the salt has a melting point of from about 209 to about 213 °C at atmospheric pressure. In more particular embodiments, the salt has a melting point of from about 210 to about 212 °C, such as about 211 °C.

When used herein in relation to a specific value (such as an amount), the term "about" (or similar terms, such as "approximately") will be understood as indicating that such values may vary by up to 10% (particularly, up to 5%, such as up to 1%) of the value defined. It is contemplated that, at each instance, such terms may be replaced with the notation "±10%", or the like (or by indicating a variance of a specific amount calculated based on the relevant value). It is also contemplated that, at each instance, such terms may be deleted.

In certain embodiments, the salt is crystalline (i.e. a crystalline solid; when observed at room temperature, e.g. 20 °C, and atmospheric pressure).

The skilled person will appreciate that the compound (salt) of the invention is crystalline and therefore may exist as a particular crystalline form. The skilled person will be familiar with techniques that may be used to characterise such crystalline forms, such as X-ray powder diffraction (XRPD).

In particular embodiments, the salt is characterised by X-ray powder diffraction peaks at 2Θ angles comprising peaks at 11.35 17.24 and 18.19.

In more particular embodiments, the salt is characterised by X-ray powder diffraction peaks at 2Θ angles comprising peaks at 11.35, 17.24, 18.19 and 18.91.

In more particular embodiments, the salt is characterised by X-ray powder diffraction peaks at 2Θ angles comprising peaks at 11.35, 17.24, 18.19, 18.91 and 22.80.

In particular embodiments, the salt is characterised by at least three X-ray powder diffraction peaks at 2Θ angles selected from those indicated in Table A (below):

**Table A**

| **Peak number** | **2θ [°]** |
|---|---|
| 1 | 5.74 |
| 2 | 11.35 |
| 3 | 14.04 |
| 4 | 14.64 |
| 5 | 17.24 |
| 6 | 18.19 |
| 7 | 18.91 |
| 8 | 20.04 |
| 9 | 21.21 |
| 10 | 22.01 |
| 11 | 22.80 |
| 12 | 24.96 |
| 13 | 25.65 |
| 14 | 26.53 |
| 15 | 27.66 |
| 16 | 28.12 |
| 17 | 30.09 |
| 18 | 31.41 |
| 19 | 32.05 |
| 20 | 32.83 |

In more particular embodiments, the salt is characterised by having at least four, such as at least five, including at least six, seven, eight or nine (or, particularly, at least 10) X-ray powder diffraction peaks at 2Θ angles selected from those in Table A.

In even more particular embodiments, the salt is characterised by having X-ray powder diffraction peaks at 2Θ angles comprising peaks corresponding (or substantially corresponding) to those in Table A.

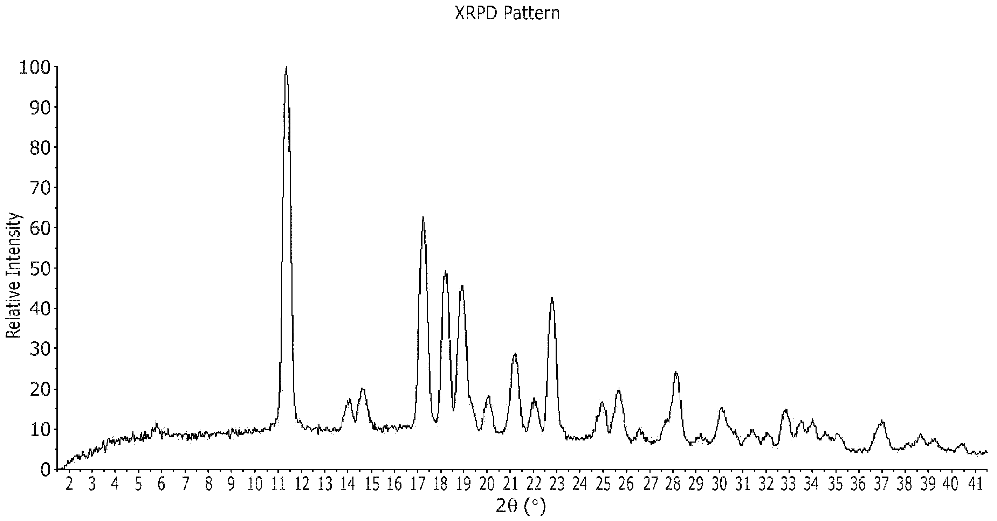

In certain embodiments, the salt is characterised by having an X-ray powder diffraction pattern substantially according to that shown in Figure 1.

In certain embodiments, the salt is a solvate. By "solvate", we mean that the salt contains molecules of solvent inside the crystalline structure, which solvents may be those used in the preparation thereof (e.g. i-PrOH, EtOH or a mixture of EtOH and water; see the examples provided herein).

In more particular embodiments, the salt is a hydrate. By "hydrate", we mean that the salt contains molecules of water inside the crystalline structure.

In certain embodiments, the salt may be described as substantially non-hygroscopic or non-hygroscopic, which terms will be understood by those skilled in the art. In particular, the term substantially non-hygroscopic may be taken to mean that after exposure to conditions of 75% relative humidity at 40 °C for 2 days, the salt absorbs 0.5 wt.% or less of water (such as less than 0.3 wt.% or 0.2 wt.% water, more particularly 0.1 wt.% or less of water).

### Medical uses

As indicated herein, the compounds of the invention, and therefore compositions and kits comprising the same, are useful as pharmaceuticals.

Thus, according to a second aspect of the invention there is provided a salt of the first aspect of the invention, as hereinbefore defined (i.e. a salt as defined in the first aspect of the invention, including all embodiments and particular features thereof), for use as a pharmaceutical (or for use in medicine).

As indicated herein, the salts of the invention may be of particular use in treating hyperglycaemia or a disorder characterized by hyperglycaemia.

Thus, in a third aspect of the invention, there is provided a salt of the first aspect of the invention, as hereinbefore defined, for use in the treatment of hyperglycaemia or a disorder characterized by hyperglycaemia.

For the avoidance of doubt, the term "hyperglycaemia" as used herein will be understood by those skilled in the art to refer to a condition wherein an excessive amount of glucose circulates in blood plasma of the subject experiencing the same. In particular, it may refer to a subject (e.g a human subject) having blood glucose levels higher than about 10.0 mmol/L (such as higher than about 11.1 mmol/L, e.g. higher than about 15 mmol/L), although it may also refer to a subject (e.g a human subject) having blood glucose levels higher than about 7 mmol/L for an extended period of time (e.g. for greater than 24 hours, such as for greater than 48 hours).

The skilled person will understand that references to the treatment of a particular condition (or, similarly, to treating that condition) take their normal meanings in the field of medicine. In particular, the terms may refer to achieving a reduction in the severity of one or more clinical symptom associated with the condition. For example, in the case of type 2 diabetes, the term may refer to achieving a reduction of blood glucose levels. In particular embodiments, in the case of treating hyperglycaemia or conditions characterised by hyperglycaemia, the term may refer to achieving a reduction of blood glucose levels (for example, to or below about 10.0 mmol/mL (e.g. to levels in the range of from about 4.0 mmol/L to about 10.0 mmol/L), such as to or below about 7.5 mmol/mL (e.g. to levels in the range of from about 4.0 mmol/L to about 7.5 mmol/L) or to or below about 6 mmol/mL (e.g. to levels in the range of from about 4.0 mmol/L to about 6.0 mmol/L)).

As used herein, references to patients will refer to a living subject being treated, including mammalian (e.g. human) patients. Thus, in particular embodiments of the first aspect of the invention, the treatment is in a mammal (e.g. a human).

As used herein, the term therapeutically effective amount will refer to an amount of a salt that confers a therapeutic effect on the treated patient. The effect may be objective (i.e. measurable by some test or marker) or subjective (i.e. the subject gives an indication of and/or feels an effect).

For the avoidance of doubt, the compounds of the first aspect of the invention are useful because they possess pharmacological activity, and/or are metabolised in the body following oral or parenteral administration to form compounds that possess pharmacological activity. In particular, as described herein, compounds of the first aspect of the invention are useful in the treatment of hyperglycaemia or disorders characterized by hyperglycaemia (such as type 2 diabetes), which terms will be readily understood by one of skill in the art (as described herein).

In a particular embodiment, the treatment is of a disorder (which may also be referred to as a condition or disease) characterised by hyperglycaemia.

In particular embodiments, salts of the invention (i.e. hemi-tartrate salts of the compound of formula I, including all embodiments thereof) are for use in the treatment of type 2 diabetes (or useful in the manufacture of a medicament for such treatment, or useful in a method for such treatment, as described herein).

In particular embodiments of the first aspect of the invention, the disorder is type 2 diabetes, such as type 2 diabetes of a sub-type selected from the list consisting of maturity-onset diabetes in the young (MODY), ketosis-prone diabetes in adults, latent autoimmune diabetes of adults (LADA), and gestational diabetes.

In further particular embodiments, the treatment of type 2 diabetes is in a non-obese patient.

For the avoidance of doubt, the skilled person will understand that patients with a Body Mass Index (BMI) of greater than 30 are considered to be obese.

In particular embodiments, the treatment may be of hyperglycaemia in a patient who is at risk of developing type 2 diabetes, which condition may be defined as pre-diabetes. Thus, salts of the invention may be useful in the prevention of type 2 diabetes (e.g. in a patient having pre-diabetes).

As used herein, the term prevention (and, similarly, preventing) includes references to the prophylaxis of the disease or disorder (and vice-versa). As such, references to prevention may also be references to prophylaxis, and vice versa. In particular, the term may refer to achieving a reduction in the likelihood of the patient (or healthy subject) developing the condition (for example, at least a 10% reduction, such as at least a 20%, 30% or 40% reduction, e.g. at least a 50% reduction).

In more particular embodiments, the type 2 diabetes is characterised by the patient displaying severe insulin resistance (SIR).

In further embodiments, the treatment may be of hyperglycaemia in a patient having type 1 diabetes. Thus, salts of the invention may be useful in the treatment of hyperglycaemia in type 1 diabetes.

The skilled person will understand that salts of the invention may be useful in treating hyperglycaemia in patients having impaired insulin production, such as in patients having cystic fibrosis. Thus, in further embodiments, the disorder characterized by hyperglycaemia is cystic fibrosis-related diabetes.

In particular embodiments that may be mentioned, the disorder characterised by hyperglycaemia is (or is characterized by) severe insulin resistance (SIR), which may be understood by those in the art to refer to disorders wherein typically the subject has normal, or in some cases increased, insulin production but significantly reduced insulin sensitivity. In particular instances, such patients may be non-obese (e.g. being of a healthy weight). Thus, in particular embodiments, such treatments are performed in patients who are not defined as being obese (e.g. in patients who are defined as being of a healthy weight).

For example, SIR may be identified in a patient based in said patient having fasting insulin >150 pmol/L and/or a peak insulin on glucose tolerance testing of >1,500 pmol/L, particularly in individuals with a BMI < 30kg/m² (which patient may otherwise have normal glucose tolerance).

More particularly, SIR may be characterised by the patient having no significant response to the presence of insulin, which may result from a defect (e.g. a genetic defect) in the function of the insulin receptor.

Particular disorders that may be characterised by SIR include: Rabson-Mendenhall syndrome, Donohue's syndrome (leprechaunism), Type A and Type B syndromes of insulin resistance, the HAIR-AN (hyperandrogenism, insulin resistance, and acanthosis nigricans) syndromes, pseudoacromegaly, and lipodystrophy.

More particular disorders that may be characterised by SIR include Donohue's syndrome and Type A syndrome of insulin resistance and, yet more particularly, Rabson-Mendenhall syndrome.

The skilled person will understand that treatment with salts of the first aspect of the invention may further comprise (i.e. be combined with) further (i.e. additional/other) treatment(s) for the same condition. In particular, treatment with compounds of the invention may be combined with other means for the treatment of type 2 diabetes, such as treatment with one or more other therapeutic agent that is useful in the treatment of type 2 diabetes as known to those skilled in the art, such as therapies comprising requiring the patient to undergo a change of diet and/or undertake exercise regiments, and/or surgical procedures designed to promote weight loss (such as gastric band surgery).

In particular, treatment with salts of the invention may be performed in combination with (e.g. in a patient who is also being treated with) one or more (e.g. one) additional compounds (i.e. therapeutic agents) that:
(i) are capable of reducing blood sugar levels; and/or
(ii) are insulin sensitizers; and/or
(iii) enhance insulin release,
all of which are described herein below.

In alternative embodiments, salts of the first aspect of the invention (i.e. salts of the invention) may be useful in the treatment of a non-alcoholic fatty liver disease (NAFLD).

Non-alcoholic fatty liver disease (NAFLD) is defined by excessive fat accumulation in the form of triglycerides (steatosis) in the liver (designated as an accumulation of greater than 5% of hepatocytes histologically). It is the most common liver disorder in developed countries (for example, affecting around 30% of US adults) and most patients are asymptomatic. If left untreated, the condition may progressively worsen and may ultimately lead to cirrhosis of the liver. NAFLD is particularly prevalent in obese patients, with around 80% thought to have the disease.

A sub-group of NAFLD patients (for example, between 2 and 5% of US adults) exhibit liver cell injury and inflammation in addition to excessive fat accumulation. This condition, designated as non-alcoholic steatohepatitis (NASH), is virtually indistinguishable histologically from alcoholic steatohepatitis. While the simple steatosis seen in NAFLD does not directly correlate with increased short-term morbidity or mortality, progression of this condition to NASH dramatically increases the risks of cirrhosis, liver failure and hepatocellular carcinoma. Indeed, NASH is now considered to be one of the main causes of cirrhosis (including cryptogenic cirrhosis) in the developed world.

The exact cause of NASH has yet to be elucidated, and it is almost certainly not the same in every patient. It is most closely related to insulin resistance, obesity, and the metabolic syndrome (which includes diseases related to diabetes mellitus type 2, insulin resistance, central (truncal) obesity, hyperlipidaemia, low high-density lipoprotein (HDL) cholesterol, hypertriglyceridemia, and hypertension). However, not all patients with these conditions have NASH, and not all patients with NASH suffer from one of these conditions. Nevertheless, given that NASH is a potentially fatal condition, leading to cirrhosis, liver failure and hepatocellular carcinoma, there exists a clear need for an effective treatment.

In particular embodiments, there is provided a salt of the invention (i.e. hemi-tartrate salts of the compound of formula I, including all embodiments thereof) for use in the treatment of a non-alcoholic fatty liver disease (or for use in the manufacture of a medicament for such treatment).

The process by which the triglyceride fat accumulates in liver cells is called steatosis (i.e. hepatic steatosis). The skilled person will understand that the term "steatosis" encompasses the abnormal retention of fat (i.e. lipids) within a cell. Thus, in particular embodiments of the first aspect of the invention, the treatment or prevention is of a fatty liver disease which is characterized by steatosis.

During steatosis, excess lipids accumulate in vesicles that displace the cytoplasm of the cell. Over time, the vesicles can grow large enough to distort the nucleus, and the condition is known as macrovesicular steatosis. Otherwise, the condition may be referred to as microvesicular steatosis. Steatosis is largely harmless in mild cases; however, large accumulations of fat in the liver can cause significant health issues. Risk factors associated with steatosis include diabetes mellitus, protein malnutrition, hypertension, obesity, anoxia, sleep apnea and the presence of toxins within the cell.

As described herein, fatty liver disease is most commonly associated with alcohol or a metabolic syndrome (for example, diabetes, hypertension, obesity or dyslipidemia). Therefore, depending on the underlying cause, fatty liver disease may be diagnosed as alcohol-related fatty liver disease or non-alcoholic fatty liver disease (NAFLD).

Particular diseases or conditions that are associated with fatty liver disease that are not related to alcohol include metabolic conditions such as diabetes, hypertension, obesity, dyslipidemia, abetalipoproteinemia, glycogen storage diseases, Weber-Christian disease, acute fatty liver of pregnancy, and lipodystrophy. Other non-alcohol related factors related to fatty liver diseases include malnutrition, total parenteral nutrition, severe weight loss, refeeding syndrome, jejunoileal bypass, gastric bypass, polycystic ovary syndrome and diverticulosis.

The salts of the invention have been found to be particularly useful in the treatment or prevention of NAFLD, which may be referred to as a fatty liver disease which is not alcohol related. A fatty liver disease which is "not alcohol related" may be diagnosed wherein alcohol consumption of the patient is not considered to be a main causative factor. A typical threshold for diagnosing a fatty liver disease as "not alcohol related" is a daily consumption of less than 20 g for female subjects and less than 30 g for male subjects.

If left untreated, subjects suffering from fatty liver disease may begin to experience inflammation of the the liver (hepatitis). It has been postulated that one of the possible causes of this inflammation may be lipid peroxidative damage to the membranes of the liver cells. Inflammation of a fatty liver can lead to a number of serious conditions and it is therefore desirable to treat or prevent fatty liver disease before inflammation occurs. Thus, in particular embodiments of the first aspect of the invention, the treatment or prevention is of a NAFLD which is associated with inflammation.

Non-alcoholic steatohepatitis (NASH) is the most aggressive form of NAFLD, and is a condition in which excessive fat accumulation (steatosis) is accompanied by inflammation of the liver. If advanced, NASH can lead to the development of scar tissue in the liver (fibrosis) and, eventually, cirrhosis. As described above, the compounds of the invention have been found to be useful in the treatment or prevention of NAFLD, particularly when accompanied by inflamation of the liver. It follows that the salts of the invention are also useful in the treatment or prevention of NASH. Therefore, in a further embodiment of the first aspect of the invention, the treatment or prevention is of non-alcoholic steatohepatitis (NASH).

The skilled person will understand that treatment with salts of the first aspect of the invention may further comprise (i.e. be combined with) further (i.e. additional/other) treatment(s) for the same condition. In particular, treatment with salts of the invention may be combined with other means for the treatment of a fatty liver disease, as described herein, such as treatment with one or more other therapeutic agent that is useful in the treatment of a fatty liver disease as known to those skilled in the art; for example, therapies comprising requiring the patient to undergo a change of diet and/or undertake exercise regiments, and/or surgical procedures designed to promote weight loss (such as gastric band surgery).

In particular, treatment with salts of the invention may be performed in combination with (e.g. in a patient who is also being treated with) one or more (e.g. one) additional compounds (i.e. therapeutic agents) that are capable of reducing the level of fat (e.g. triglycerides) in the liver.

References to treatment of a fatty liver disease may refer to achieving a therapeutically significant reduction of fat (e.g. triglycerides levels) in liver cells (such as a reduction of at least 5% by weight, e.g. a reduction of at least 10%, or at least 20% or even 25%).

### Pharmaceutical compositions

As described herein, salts of the first aspect of the invention are useful as pharmaceuticals. Such salts may be administered alone or may be administered by way of known pharmaceutical compositions/formulations.

In a fourth aspect of the invention, there is provided a pharmaceutical composition comprising a salt as defined in the first aspect of the invention (i.e. a salt of the invention), and optionally one or more pharmaceutically acceptable adjuvant, diluent and/or carrier.

The skilled person will understand that references herein to salts of the first aspect of the invention being for particular uses (and, similarly, to uses and methods of use relating to salts of the invention) may also apply to pharmaceutical compositions comprising salts of the invention as described herein.

In a fifth aspect of the invention, there is provided a pharmaceutical composition for use in the treatment of hyperglycaemia or a disorder characterized by hyperglycaemia (as defined herein, such as type 2 diabetes) comprising a salt as defined in the first aspect of the invention, and optionally one or more pharmaceutically acceptable adjuvant, diluent and/or carrier.

In an alternative fifth aspect of the invention, there is provided a pharmaceutical composition for use in the treatment or prevention of a non-alcoholic fatty liver disease, as defined herein.

The skilled person will understand that salts of the first (and, therefore, second and third) aspect of the invention may act systemically and/or locally (i.e. at a particular site).

The skilled person will understand that salts and compositions as described in the first to fifth aspects of the invention will normally be administered orally, intravenously, subcutaneously, buccally, rectally, dermally, nasally, tracheally, bronchially, sublingually, intranasally, topically, by any other parenteral route or *via* inhalation, in a pharmaceutically acceptable dosage form. Pharmaceutical compositions as described herein will include compositions in the form of tablets, capsules or elixirs for oral administration, suppositories for rectal administration, sterile solutions or suspensions for parenteral or intramuscular administration, and the like. Alternatively, particularly where such salts of the invention act locally, pharmaceutical compositions may be formulated for topical administration.

Thus, in particular embodiments of the fourth and fifth aspects of the invention, the pharmaceutical formulation is provided in a pharmaceutically acceptable dosage form, including tablets or capsules, liquid forms to be taken orally or by injection, suppositories, creams, gels, foams, inhalants, intranasal dosage forms, or forms suitable for topical administration. For the avoidance of doubt, in such embodiments, salts of the invention may be present as a solid (e.g. a solid dispersion), liquid (e.g. in solution) or in other forms, such as in the form of micelles.

For example, in the preparation of pharmaceutical formulations for oral administration, the salt may be mixed with solid, powdered ingredients such as lactose, saccharose, sorbitol, mannitol, starch, amylopectin, cellulose derivatives, gelatin, or another suitable ingredient, as well as with disintegrating agents and lubricating agents such as magnesium stearate, calcium stearate, sodium stearyl fumarate and polyethylene glycol waxes. The mixture may then be processed into granules or compressed into tablets.

Soft gelatin capsules may be prepared with capsules containing one or more active compounds (e.g. salts of the first and, therefore, second and third aspects of the invention, and optionally additional therapeutic agents), together with, for example, vegetable oil, fat, or other suitable vehicle for soft gelatin capsules. Similarly, hard gelatine capsules may contain such compound(s) in combination with solid powdered ingredients such as lactose, saccharose, sorbitol, mannitol, potato starch, corn starch, amylopectin, cellulose derivatives or gelatin.

Dosage units for rectal administration may be prepared (i) in the form of suppositories which contain the compound(s) mixed with a neutral fat base; (ii) in the form of a gelatin rectal capsule which contains the active substance in a mixture with a vegetable oil, paraffin oil, or other suitable vehicle for gelatin rectal capsules; (iii) in the form of a ready-made micro enema; or (iv) in the form of a dry micro enema formulation to be reconstituted in a suitable solvent just prior to administration.

Liquid preparations for oral administration may be prepared in the form of syrups or suspensions, e.g. solutions or suspensions, containing the compound(s) and the remainder of the formulation consisting of sugar or sugar alcohols, and a mixture of ethanol, water, glycerol, propylene glycol and polyethylene glycol. If desired, such liquid preparations may contain colouring agents, flavouring agents, preservatives, saccharine and carboxymethyl cellulose or other thickening agent. Liquid preparations for oral administration may also be prepared in the form of a dry powder to be reconstituted with a suitable solvent prior to use.

Solutions for parenteral administration may be prepared as a solution of the compound(s) in a pharmaceutically acceptable solvent. These solutions may also contain stabilizing ingredients and/or buffering ingredients and are dispensed into unit doses in the form of ampoules or vials. Solutions for parenteral administration may also be prepared as a dry preparation to be reconstituted with a suitable solvent extemporaneously before use.

The skilled person will understand that salts of the invention may be administered (for example, as formulations as described hereinabove) at varying doses, with suitable doses being readily determined by one of skill in the art. Oral, pulmonary and topical dosages (and subcutaneous dosages, although these dosages may be relatively lower) may range from between about 0.01 µg/kg/day of body weight per day (µg/kg/day) to about 20 mg/kg/day of body weight per day (mg/kg/day), preferably about 0.1 µg/kg/day to about 5 mg/kg/day, and more preferably about 1 µg/kg/day to about 2 mg/kg/day (e.g. about 10 µg/kg/day to about 1 mg/kg/day). For example, when administered orally, treatment with such salts may comprise administration of a formulations typically containing between about 1 µg to about 2000 mg, for example between about 10 µg to about 500 mg, or between 100 µg to about 200 mg (e.g. about 1 mg to about 100 mg), of the active ingredient(s). When administered intravenously, the most preferred doses will range from about 0.001 to about 10 µg/kg/hour during constant rate infusion. Advantageously, treatment may comprise administration of such salts and compositions in a single daily dose, or the total daily dosage may be administered in divided doses of two, three or four times daily (e.g. twice daily with reference to the doses described herein, such as a dose of 10 mg, 20 mg, 30 mg or 40 mg twice daily, or 10 µg, 20 µg, 30 µg or 40 µg twice daily).

In any event, the skilled person (e.g. the physician) will be able to determine the actual dosage which will be most suitable for an individual patient, which is likely to vary with the route of administration, the type and severity of the condition that is to be treated, as well as the species, age, weight, sex, renal function, hepatic function and response of the particular patient to be treated. The above-mentioned dosages are exemplary of the average case; there can, of course, be individual instances where higher or lower dosage ranges are merited, and such are within the scope of this invention.

As described herein above, the skilled person will understand that treatment with salts of the first aspect of the invention may further comprise (i.e. be combined with) further (i.e. additional/other) treatment(s) for the same condition. In particular, treatment with salts of the invention may be combined with other means for the treatment of hyperglycaemia or a disorder characterized by hyperglycaemia (as defined herein, such as type 2 diabetes), such as treatment with one or more other therapeutic agent that is useful in the treatment of hyperglycaemia or a disorder characterized by hyperglycaemia(as defined herein, such as type 2 diabetes).

In particular embodiments of the fourth and fifth aspects of the invention, the pharmaceutical composition may further comprise one or more additional (i.e. other) therapeutic agent.

In more particular embodiments, the one or more additional therapeutic agent is an agent for the treatment of type 2 diabetes as known to those skilled in the art, such as metformin, sulfonylureas (e.g. carbutamide, acetohexamide, chlorpropamide, tolbutamide. glipizide (glucotrol), gliclazide, glibenclamide, glyburide (Micronase), glibornuride, gliquidone, glisoxepide, glyclopyramide, glimepiride (Amaryl), glimiprime, JB253 or JB558), thiazolidinediones (e.g. pioglitazone, rosiglitazone (Avandia), lobeglitazone (Duvie) and troglitazone (Rezulin)), dipeptidyl peptidase-4 inhibitors (e.g. sitagliptin, vildagliptin, saxagliptin, linagliptin, anagliptin, teneligliptin, alogliptin, trelagliptin, gemigliptin, dutogliptin and omarigliptin), SGLT2 inhibitors (e.g. dapagliflozin, empagliflozin, canagliflozin, ipragliflozin, tofogliflozin, sergliflozin etabonate, remogliflozin etabonate, and ertugliflozin), and glucagon-like peptide-1 (GLP-1) analogues.

The skilled person will understand that combinations of therapeutic agents may also be described as a combination product and/or provided as a kit-of-parts.

In a sixth aspect of the invention, there is provided a combination product comprising:
(A) a salt as defined in the first aspect of the invention; and
(B) one or more additional therapeutic agent,
wherein each of components (A) and (B) is formulated in admixture, optionally with one or more a pharmaceutically-acceptable adjuvant, diluent or carrier.

In a seventh aspect of the invention, there is provided a kit-of-parts comprising:
(a) a salt as defined in the first (or second and/or third) aspect of the invention, (or a pharmaceutical composition comprising the same) or a pharmaceutical composition as defined in the fourth or fifth aspect of the invention; and
(b) one or more other therapeutic agent, optionally in admixture with one or more pharmaceutically-acceptable adjuvant, diluent or carrier,
which components (a) and (b) are each provided in a form that is suitable for administration in conjunction with the other.

In particular embodiments (e.g. of the sixth and seventh aspects of the invention), the additional therapeutic agent is a therapeutic agent that is useful for the treatment of hyperglycaemia or a disorder characterized by hyperglycaemia (e.g. type 2 diabetes), as known to those skilled in the art (such as those described herein).

For example, in particular embodiments of the fourth to fifth aspects of the invention, the additional therapeutic agent is an agent that:
(i) is capable of reducing blood sugar levels; and/or
(ii) is an insulin sensitizer; and/or
(iii) is able to enhance insulin release,
which agents will be readily identified by those skilled in the art and include, in particular, such therapeutic agents that are commercially available (e.g. agents that the subject of a marketing authorization in one or more territory, such as a European or US marketing authorization).

The skilled person will understand that references to therapeutic agents capable of reducing blood glucose levels may refer to compounds capable of reducing levels of blood by at least 10% (such as at least 20%, at least 30% or at least 40%, for example at least 50%, at least 60%, at least 70% or at least 80%, e.g. at least 90%) when compared to the blood glucose levels prior to treatment with the relevant compound.

In alternative embodiments of the sixth and seventh aspects of the invention, the additional therapeutic agent is an agent for the treatment or prevention of a non-alcoholic fatty liver disease (such as NASH), which agents will be readily identified by those skilled in the art and include, in particular, such therapeutic agents that are commercially available (e.g. agents that the subject of a marketing authorization in one or more territory, such as a European or US marketing authorization).

### Preparation of salts/compositions

Pharmaceutical compositions/formulations, combination products and kits as described herein may be prepared in accordance with standard and/or accepted pharmaceutical practice.

Thus, in a further aspect of the invention there is provided a process for the preparation of a pharmaceutical composition/formulation, as hereinbefore defined, which process comprises bringing into association a salt of the invention, as hereinbefore defined, with one or more pharmaceutically-acceptable adjuvant, diluent or carrier.

In further aspects of the invention, there is provided a process for the preparation of a combination product or kit-of-parts as hereinbefore defined, which process comprises bringing into association a salt of the invention, as hereinbefore defined, with the other therapeutic agent that is useful in the treatment of hyperglycaemia or a disorder characterized by hyperglycaemia (e.g. type 2 diabetes), and at least one pharmaceutically-acceptable adjuvant, diluent or carrier.

As used herein, references to bringing into association will mean that the two components are rendered suitable for administration in conjunction with each other.

Thus, in relation to the process for the preparation of a kit of parts as hereinbefore defined, by bringing the two components "into association with" each other, we include that the two components of the kit of parts may be:
(i) provided as separate formulations (i.e. independently of one another), which are subsequently brought together for use in conjunction with each other in combination therapy; or
(ii) packaged and presented together as separate components of a "combination pack" for use in conjunction with each other in combination therapy.

### Preparation of compounds and salts

The compounds as defined in the first (and, therefore, second and third) aspect of the invention (i.e. the compound of formula I) may be prepared in accordance with techniques that are well known to those skilled in the art, such as those described in the examples provided hereinafter. For example, the compound of formula I may be made in accordance with the techniques described in international patent application WO 2019/053427, the content of which (in particular, the examples) is hereby incorporated by reference.

The salts of the first (and, therefore, second and third) aspect of the invention (i.e. the salts of the invention) may be prepared in accordance with techniques that are well known to those in the art, such as those described in the examples provided hereinafter. For example, the compound of formula I may be reacted with tartaric acid or a solution of tartaric acid (or vice versa). Salt switching techniques may also be used to convert one salt into another salt.

In certain embodiments, the salt of the invention is prepared by reacting a compound of formula I with a solution of tartaric acid (or vice versa).

In particular embodiments that may be mentioned, the solvent in the solution is ethanol or an ethanol-water mixture. The use of ethanol or an ethanol-water mixture as the solvent allows the scaling up of the manufacture of the hemi-tartrate salt of the compound of formula I with a high yield, high purity and high enantiomeric purity.

Salts as described herein (in particular, salts as defined in the first and, therefore, second and third aspects of the invention) may have the advantage that they may be more efficacious than, be less toxic than, be longer acting than, be more potent than, produce fewer side effects than, be more easily absorbed than, and/or have a better pharmacokinetic profile (e.g. higher oral bioavailability and/or lower clearance) than, and/or have other useful pharmacological, physical, or chemical properties over, compounds known in the prior art, whether for use in the above-stated indications or otherwise. In particular, such salts may have the advantage that they are more efficacious and/or exhibit advantageous properties *in vivo.*

Without wishing to be bound by theory, compounds of the invention are thought to be potent agonists of the β₂-adrenergic receptor, which allows for increased glucose uptake in skeletal muscle cells. Compounds of the invention are also believed to provide suitable levels of metabolic stability.

In addition, salts as described herein are thought to be agonists of the β₂-adrenergic receptor without (or with only a relatively minimal effect in, such as a relatively lesser effect in (when compared to the effect in inducing increased glucose uptake)) inducing cAMP production. It is thought that this allows for the increased glucose uptake in skeletal muscle cells with lower levels of side effects than would result from other treatments. Further, combining salts as described herein with therapeutic agents that are able to decrease blood glucose levels is thought to provide an effective combination therapy.

Furthermore, salts and compositions of the invention may have a number of advantages compared to the corresponding free base (i.e. the free base of the compound of formula I). For example, the salts and compositions of the invention may have improved ease of formulation, improved stability, improved purity, improved bioavailability, improved taste, improved ability to be scaled up in manufacturing processes and/or lower hygroscopicity compared to the corresponding free base. Such salts will also ideally have low toxicity and thus be suitable for use in medicine (i.e. pharmaceutically acceptable).

More specifically, the free base of the compound of formula I is difficult to prepare as a solid, either being obtained as an oil or as a solid in low yield. Other salts of the compound of formula I either could not be crystallised, could only be crystallised in low yield and/or low purity, or formed such small crystals that filtration, isolation and subsequent scale-up of the synthesis was difficult. In contrast, it is surprising to find that that the hemi-tartrate salt of the compound of formula I may be readily obtained as a crystalline solid in high yield, with high purity (and in high enantiomeric excess) and in a form that demonstrates low hygroscopicity.

### Figures

Figure 1 shows an XRPD analysis of the hemitartrate salt of (R)-2-(tert-butylamino)-1-(5-fluoropyridin-3-yl)ethan-1-ol.

### Examples

The present invention is illustrated by way of the following examples.

Chemicals and reagents were obtained from commercial suppliers and were used as received unless otherwise stated. All reactions involving moisture sensitive reagents were performed in oven or flame dried glassware under a positive pressure of nitrogen or argon.

### Abbreviations

Abbreviations as used herein will be known to those skilled in the art. In particular, the following abbreviations may be used herein.
- aq: aqueous
- Et₂O: diethylether
- EtOAc: ethyl acetate
- EtOH: ethanol
- iPrOAc: isopropyl acetate
- iPrOH: isopropanol
- MeCN: acetonitrile
- rt: room temperature
- THF: tetrahydrofuran

### Example compounds and salts

In the event that there is a discrepancy between nomenclature and the structure of compounds as depicted graphically, it is the latter that presides (unless contradicted by any experimental details that may be given and/or unless it is clear from the context).

### Compound Example: (R)-2-(tert-Butylamino)-1-(5-fluoropyridin-3-yl)ethan-1-ol

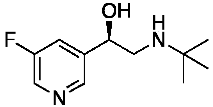

### (a) 2-Chloro-1-(5-fluoropyridin-3-yl)ethan-1-one

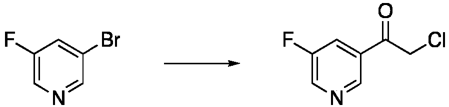

Isopropylmagnesium chloride (2 M in THF, 10.47 mL, 20.94 mmol) was added to a solution of LiCl (887.69 mg, 20.94 mmol) in THF (8 mL) at rt. After 15 min at rt, 3-bromo-5-fluoropyridine (3.35 g, 19.04 mmol) in THF (30 mL) was added dropwise at 0 °C. The mixture was stirred at rt for 2 h and cooled in an ice-bath. A solution of 2-chloro-N-methoxy-N-methylacetamide (2.62 g, 19.04 mmol) in THF (30 mL) was added dropwise, and the mixture was stirred at rt for 2 h. NH₄Cl (aq, 10 %) was added and the mixture was extracted with Et₂O. The combined extracts were washed with brine, dried (Na₂SO₄) and concentrated. The residue was purified by chromatography to give the sub-title compound (1.52 g, 20.94 mmol, 46 %).

### (b) (R)-2-Chloro-1-(5-fluoropyridin-3-yl)ethan-1-one

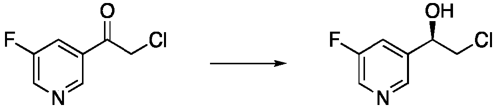

iPrOAc (250 mL, 10 vol), pentamethylcyclopentadienylrhodium(III) chloride dimer (74 mg, 0.001 eq), (1S,2S)-(+)-N-*p*-tosyl-1,2-diphenylethylenediamine (87 mg, 0.002 eq) and NEt₃ (66 µL, 0.004 eq) were stirred at rt for 1h20, before 2-chloro-1-(5-fluoropyridin-3-yl)ethan-1-one hydrochloride (25g, 1.00 eq) was added. The mixture was cooled to 7 °C, NEt₃ (83 mL, 5.00 eq) was added and then HCOOH (9 mL, 2.00 eq) was added. The mixture was stirred at rt for 1h, before being diluted with 0.5 M aq HCl, extracted with iPrOAc, washed with H₂O and concentrated. The residue was diluted with iPrOH, cooled to 0 °C and then 5.56 M HCl in iPrOH (22 mL, 1.00 eq) was added. The solids were collected to give the sub-title compound (18.08g, 72%, ee = 98.7%).

### (c) (R)-2-(tert-Butylamino)-1-(5-fluoropyridin-3-yl)ethan-1-ol

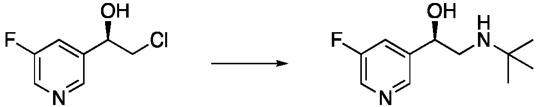

tert-Butylamine (11.37 mL, 108.21 mmol) followed by NaOH (476.08 mg, 11.90 mmol) were added to a mixture of 2-chloro-1-(5-fluoropyridin-3-yl)ethan-1-one (1.90 g, 10.82 mmol) and iPrOH (1.66 mL, 21.64 mmol) at rt. The mixture was heated at 75 °C for 4 h, allowed to cool, diluted with EtOAc, washed with H₂O and brine, dried (Na₂SO₄) and concentrated. The residue was dissolved in hot EtOAc and allowed to cool. Pentane was added and the mixture kept at -20 °C overnight. The solids were collected and purified by chromatography to give the title compound (1.43 g, 6.74 mmol, 62 %, ee = 98 %). ¹H NMR (400 MHz, CDCl₃): δ 8.43 - 8.27 (m, 2H), 7.57 - 7.42 (m, 1H), 4.62 (dd, J = 8.8, 3.7 Hz, 1H), 2.94 (dd, J = 12.1, 3.8 Hz, 1H), 2.53 (dd, J = 12.1, 8.8 Hz, 1H), 1.10 (s, 9H).

In the above-described experiments, the title compound was isolated as the free base by evaporation to dryness. However, this isolation method typically conducted at laboratory scale. Therefore, different solvents and solvent mixtures were tested to crystallize the free base. Surprisingly, all initial attempts resulted in either formation of crystals with relatively low yield (below 50%) or oil separation. It was therefore decided to investigate salt formation.

### Salt Example 1: Hemi-tartrate

A solution of L-(+)-tartaric acid (6.21g, 0.5 eq) in EtOH (175 mL) was added to a solution of (*R*)-2-(*tert*-butylamino)-1-(5-fluoropyridin-3-yl)ethan-1-ol (17.57 g) in EtOH (525 mL, 30 vol) and H₂O (7 mL) at rt. The mixture was refluxed until all precipitate was dissolved, then cooled. The resultant slurry was stirred at rt overnight and then at 0 to 5 °C for 2 h. The solids were collected to give the title salt of (R)-2-(tert-butylamino)-1-(5-fluoropyridin-3-yl)ethan-1-ol (19.9 g, 83%, 100.0% purity by HPLC, 99.8% ee by HPLC).

Formation of the hemitartrate was confirmed by ¹H-NMR spectrum, which indicated an amine:acid ratio of 2:1.

Characterization of solid-state properties of the title salt using XRPD, DSC and DVS techniques indicated that material is crystalline with a high melting point at 211 °C. The salt was not hygroscopic and was physically stable under the tested conditions (2 days at 40 °C and 75% RH).

It was observed during the experiment that filtration of the hemitartrate salt was very good, and so further experiments were carried out to optimise the conditions for the formation of the hemitartrate salt at increased scale. The results are shown in the table below.

| **Batch** | **Scale, 9** | **Conditions** | **Used base:acid ratio, eq** | **Yield, %** | **Purity, HPLC (area-%)** | **Comments** |
|---|---|---|---|---|---|---|
| 1a | 1.2 | EtOH (30 vol) rt → + 5°C | 1: 1 | 65 | 99.9 | Thick slurry was formed. Mixture was diluted with additional 5 vol of EtOH to improve mixing and then filtered. |
| 1b | 2.0 | EtOH/H₂O (0.6 vol-%; 42 vol) rt → reflux → RT → + 5°C | 1:0.6 | 71 | 99.9 | Thick slurry was formed. Water 0.6 vol% was added to improve mixing. |
| 1c | 2.0 | EtOH/H₂O (1 vol-%; 30→ 40 vol) rt → reflux → rt → + 5°C | 1:0.6 | 76 | 99.8 | Full dissolution by heating was achieved only after addition of extra 10 vol of EtOH. |
| 1d | 2.0 | EtOH/H₂O (1 vol; 40 vol) 40 °C → reflux → rt → + 5°C | 1:0.5 | 84 | 99.9 | Full dissolution at reflux temperature was observed. Good mixing and filtration. |
| 1e | 5.0 | EtOH/H₂O (1 vol-%; 40 vol) rt → reflux → + 5°C | 1:0.5 | 83 | 99.9 | Full dissolution at reflux temperature. Crystallization started at 57 °C. |
| 1f | 20.0 | EtOH/H₂O (1 vol-%; 40 vol) rt → reflux → + 5°C | 1:0.5 | 83 | 100.0 | Upscale experiment. Good mixing and filterability. |

Overall, the hemitartrate salt of (*R*)-2-(*tert*-butylamino)-1-(5-fluoropyridin-3-yl)ethan-1-ol was obtained in high yield, high purity and with an enantiomeric excess over 99% for (*R*)-2-(*tert*-butylamino)-1-(5-fluoropyridin-3-yl)ethan-1-ol. In particular, use of the free base of (R)-2-(tert-butylamino)-1-(5-fluoropyridin-3-yl)ethan-1-ol having a purity of only 95.6 % (by HPLC) was used to prepare the hemitartrate salt having a purity in excess of 99.8 % (by HPLC), indicating a significant purification effect upon crystallization.

Furthermore, the synthesis could be easily upscaled and, unlike many of the other salt forms of (*R)*-2-(*tert*-butylamino)-1-(5-fluoropyridin-3-yl)ethan-1-ol, had a low hygroscopicity and did not have any problems with slow filtration, which was thought to be caused by small crystal formation.

A sample of the hemitartrate salt was analysed by X-ray powder diffraction and found to have a diffraction pattern generating peaks as shown in Figure 1. The data for the relevant peaks are summarised in the table below.

| **#** | **2θ [°]** | **d [Å]** | **Intensity** |
|---|---|---|---|
| 1 | 5.74 | 15.38 | 7 |
| 2 | 11.35 | 7.79 | 100 |
| 3 | 14.04 | 6.30 | 9 |
| 4 | 14.64 | 6.04 | 11 |
| 5 | 17.24 | 5.14 | 58 |
| 6 | 18.19 | 4.87 | 44 |
| 7 | 18.91 | 4.69 | 40 |
| 8 | 20.04 | 4.43 | 9 |
| 9 | 21.21 | 4.19 | 21 |
| 10 | 22.01 | 4.03 | 10 |
| 11 | 22.80 | 3.90 | 38 |
| 12 | 24.96 | 3.56 | 10 |
| 13 | 25.65 | 3.47 | 14 |
| 14 | 26.53 | 3.36 | 3 |
| 15 | 27.66 | 3.22 | 6 |
| 16 | 28.12 | 3.17 | 19 |
| 17 | 30.09 | 2.97 | 10 |
| 18 | 31.41 | 2.85 | 4 |
| 19 | 32.05 | 2.79 | 3 |
| 20 | 32.83 | 2.73 | 10 |

### Comparative Salt Example 2: Dihydrochloride

Aqueous hydrochloric acid (37%, 1.1 eq) was added to a solution of (*R*)-2-(*tert-*butylamino)-1-(5-fluoropyridin-3-yl)ethan-1-ol (300 mg, 1.0 eq) in iPrOH (10 vol) at 40 °C. The resultant slurry was stirred at rt overnight. The solids were collected to give the title salt of (*R)*-2-(*tert*-butylamino)-1-(5-fluoropyridin-3-yl)ethan-1-ol (47%).

The synthesis was repeated using different solvents and acids, the results of which are shown in the table below.

| **Batch** | **Scale (mg)** | **Solvent (10 vol)** | **Acid** | **Yield, (%)** | **Purity, (area-% HPLC)** |
|---|---|---|---|---|---|
| 2a | 500 | iPrOH | 5.6M HCl in iPrOH | 60 | 98.2 |
| 2b | 500 | iPrOH:iPrOAc (1:1) | 5.6M HCl in iPrOH | 84 | 94.9 |

The DSC analysis of the title salt of (R)-2-(tert-butylamino)-1-(5-fluoropyridin-3-yl)ethan-1-ol showed a broad endothermic event at ~150 °C with mass loss due to possible dehydration or desolvation and a second endothermic event at ~220 °C. Based on observations and information collected during synthesis of the dihydrochloride salt, it is supposed that the product has high tendency to form hydrate or solvate during a salt preparation. Since good yield and purity of the dihydrochloride salt was not achieved, it was decided to discontinue work on this salt form.

### Comparative Salt Example 3: Edisylate

1,2-Ethanedisulfonic acid (1.05 eq) was added to a solution of (R)-2-(tert-butylamino)-1-(5-fluoropyridin-3-yl)ethan-1-ol (500 mg, 1.0 eq) in iPrOH (20 vol) at rt. The resultant slurry was stirred at rt overnight. The solids were collected to give the title salt of (R)-2-(*tert*-butylamino)-1-(5-fluoropyridin-3-yl)ethan-1-ol.

The salt was found to be hygroscopic under the tested conditions (2 days at 40 °C and 75% RH).

It was observed during the experiment that filtration of the edisylate salt was very slow due to the formation of very fine solids.

The synthesis was repeated using different solvents and acids, the results of which are shown in the table below.

| **Batch** | **Scale (mg)** | **Conditions** | **Comment** |
|---|---|---|---|
| 3a | 500 | iPrOH, 20 vol, rt | Thick slurry was formed. Filtration was very slow. |
| 3b | 400 | 2-vol% H₂O in THF, 15 vol, rt | Slurry was formed. Moderate filtration speed. |
| 3c | 200 | EtOH, 15 vol, rt | Very fine suspension was formed. The fine solids went through the glass filter frit 3 during the filtration. |
| 3d | 200 | EtOH/H₂O (2 vol-%), 15 vol, rt | Very fine suspension was formed. The fine solids went through the glass filter frit 3 during the filtration. |
| 3e | 200 | *i*-PrOH/H₂O (2 vol-%), 15 vol, rt | Very fine suspension was formed. The fine solids went through the glass filter frit 3 during the filtration. |
| 3f | 200 | THF/H₂O (1 vol-%), 15 vol, rt | Slurry was formed. Filtration was fast. Low purity (94.7% by HPLC). |

Further development of the edisylate salt for production was not carried out due to the low purification achieved from salt formation and due to the limited commercial availability of pharmaceutical grade 1,2-ethanedisulfonic acid in bulk amounts.

### Comparative Salt Example 4: Maleate

Maleic acid (1.1 eq) was added to a solution of (R)-2-(tert-butylamino)-1-(5-fluoropyridin-3-yl)ethan-1-ol (50 mg, 1.0 eq) in iPrOH (10 vol) at 40 °C. The resultant slurry was stirred at rt overnight. The solids were collected to give the title salt of (R)-2-(*tert*-butylamino)-1-(5-fluoropyridin-3-yl)ethan-1-ol.

The salt was slightly hygroscopic under the tested conditions (2 days at 40 °C and 75% RH), with a mass loss of between 0.9 wt.% and 1.4 wt.% due to water seen between 25 and 155 °C upon subsequent TGMS analysis.

It was observed during the experiment that filtration of the maleate salt was very slow due to the formation of very fine solids.

The title salt of (R)-2-(tert-butylamino)-1-(5-fluoropyridin-3-yl)ethan-1-ol was used in recrystallisation attempts to find conditions which facilitate formation of larger particles and improved filtration. Conditions identified in screening experiments were further applied for salt formation experiments on 50 mg scale, the results of which are shown in the table below.

| **Comparative Example** | **Solvent/Volumes** | **Observations** |
|---|---|---|
| 4a | THF/1.5-vol% H₂O (30 vol) | Sticky solids formed. |
| 4b | iPrOH (30 vol) | Slow filtration. |
| 4c | iPrOH/5-vol% H₂O (10 vol) | Slow filtration. |
| 4d | iPrOH/i-PrOAc (3:1, 40 vol) | Slow filtration. |
| 4e | iPrOH/5-vol% EtOH (20 vol) | Slow filtration |
| 4f | THF/10-vol% EtOH (10 vol) | Small amount precipitated. |
| 4g | MeCN (30 vol) | Slow filtration. |
| 4h | MeCN/iPrOAc (3:1, 40 vol) | Slow filtration. |
| 4i | MeCN/5-vol% H₂O (10 vol) | Precipitation was not started. |

### Comparative Salt Example 5: Citrate

An aqueous solution of citric acid (1 M, 1.1 eq) was added to a solution of (*R*)-2-(*tert-*butylamino)-1-(5-fluoropyridin-3-yl)ethan-1-ol (40 mg, 1.0 eq) in THF/water (50:50 v:v) at rt. The resultant mixture was freeze-dried overnight. No crystallisation occurred.

Half of the mixture was dissolved in EtOAc; the other half of the mixture was dissolved in MeCN. The solutions were subjected to three temperature cycles between 5 and 50 °C of 12 h each, then left at rt for 3 days. No crystallisation occurred.

### Comparative Salt Example 6: Succinate

Succinic acid (1.0 eq) was added to a solution of (R)-2-(tert-butylamino)-1-(5-fluoropyridin-3-yl)ethan-1-ol (100 mg, 1.0 eq) in iPrOH (20 vol) at 40 °C. The resultant mixture was stirred at rt overnight. No crystallisation occurred.

### Comparative Salt Example 7: p-Toluenesulfonate

*p*-Toluenesulfonic acid (1.2 eq) was added to a solution of (R)-2-(tert-butylamino)-1-(5-fluoropyridin-3-yl)ethan-1-ol (100 mg, 1.0 eq) in i-PrOH (20 vol) at 40 °C. The resultant slurry was stirred at rt overnight. The solids were collected to give the title salt of (R)-2-(*tert*-butylamino)-1-(5-fluoropyridin-3-yl)ethan-1-ol (106%, 86.9% purity by HPLC).

It was observed during the experiment that filtration of the *p*-toluenesulfonate salt was very slow due to the formation of very fine solids.

The salt was slightly hygroscopic under the tested conditions (2 days at 40 °C and 75% RH), with a mass loss of between 1.6 wt.% and 2.1 wt.% due to water seen between 25 and 160 °C upon subsequent TGMS analysis.

### Comparative Salt Example 8: Fumarate

Fumaric acid (1.2 eq) was added to a solution of (R)-2-(tert-butylamino)-1-(5-fluoropyridin-3-yl)ethan-1-ol (100 mg, 1.0 eq) in EtOH (10 vol) at 40 °C. The resultant mixture was stirred at rt overnight. No crystallisation occurred.

The mixture was evaporate to dryness and then crystallised from iPrOH. The solids were collected to give the title salt of (*R*)-2-(*tert*-butylamino)-1-(5-fluoropyridin-3-yl)ethan-1-ol.

### Comparative Salt Example 9: Oxalate

An aqueous solution of oxalic acid (1 M, 1.1 eq) was added to a solution of (*R*)-2-(*tert-*butylamino)-1-(5-fluoropyridin-3-yl)ethan-1-ol (40 mg, 1.0 eq) in THF/water (50:50 v:v) at rt. The resultant mixture was freeze-dried overnight.

Half of the mixture was dissolved in EtOAc; the other half of the mixture was dissolved in MeCN. The solutions were subjected to three temperature cycles between 5 and 50 °C of 12 h each, then left at rt for 3 days. In each case, the solids were collected to give the title salt of (R)-2-(tert-butylamino)-1-(5-fluoropyridin-3-yl)ethan-1-ol (98.1% purity by HPLC.

Formation of the oxalate was confirmed by ¹H-NMR spectrum, which indicated an amine:acid ratio of 1:1.

Characterization of solid-state properties of the title salt using XRPD, DSC and DVS techniques indicated that material is crystalline with a high melting point at 171 °C. The salt was physically stable and was not hygroscopic under the tested conditions (2 days at 40 °C and 75% RH), showing no significant mass loss between 60 and 160 °C upon subsequent TGMS analysis.

Further scale-up of the title salt was not carried out due to concerns about potential nephrotoxicity in patients with diabetes.

### Comparative Salt Example 10: Tartrate

An aqueous solution of L-(+)-tartaric acid (1 M, 1.1 eq) was added to a solution of (R)-2-(tert-butylamino)-1-(5-fluoropyridin-3-yl)ethan-1-ol (40 mg, 1.0 eq) in THF/water (50:50 v:v) at rt. The resultant mixture was freeze-dried overnight. No crystallisation occurred.

Half of the mixture was dissolved in EtOAc; the other half of the mixture was dissolved in MeCN. The solutions were subjected to three temperature cycles between 5 and 50 °C of 12 h each, then left at rt for 3 days. No crystallisation occurred.

### Biological examples

L6-myoblasts were grown in Dulbecco's Modified Eagle's Medium (DMEM) containing 1 g/L glucose supplemented with 10 % fetal bovine serum, 2 mM L-Glutamine, 50 U/mL penicillin, 50 µg/mL streptomycin and 10 mM HEPES. Cells were plated at 1x 10⁵ cells per mL in 24- well plates. After reaching 90 % confluence the cells were grown in medium containing 2 % FBS for 7 days where upon cells differentiated into myotubes.

### Biological example 1: Glucose uptake

Differentiated L6-myotubes were serum-starved overnight in medium containing 0.5 % fatty-acid free BSA and stimulated with an agonist, with a final concentration of 1x10⁻⁵ M. After 1 h 40 min the cells were washed with warm glucose free medium or PBS twice and another portion of agonist was added to the glucose free medium. After 20 min the cells were exposed to 50 nM ³H-2-deoxyglucose for 10 min before washed with ice cold glucose free medium or PBS three times and lysed with 0.2 M NaOH, 400 µL/well, for 1 h at 60 °C. The cell lysate was mixed with 4 mL scintillation buffer (Emulsifier Safe, Perkin Elmer) and the radioactivity was detected in a β-counter (Tri-Carb 4810TR, Perkin Elmer).

The compound of Compound Example 1 showed an activity of more than 75% of that of isoproterenol.

### Biological example 2: Measurement of intracellular cAMP levels

Differentiated cells were serum-starved overnight and stimulated with an agonist, final concentration 1x10⁻⁵ M, for 15 min in stimulation buffer (HBSS supplemented with 1 % BSA, 5 mM HEPES and 1 mM IBMX, pH 7.4). The medium was aspirated and 100 µL of 95 % EtOH was added to each well of the 24-well plate and cells were kept at -20 °C overnight. The EtOH was allowed to evaporate and 500 µL of lysis buffer (1 % BSA, 5 mM HEPES and 0.3 % Tween- 20, pH 7.4) was added to each well. The plate was kept at -80 °C for 30 min and then at -20 °C until the day of detection when the samples were thawed. Intracellular cAMP levels were detected using an alpha screen cAMP kit (6760635D from Perkin Elmer).

The compound of Compound Example 1 showed an activity of less than 50% of that of isoproterenol.

## Claims

1. A hemi-tartrate salt of a compound of formula I:

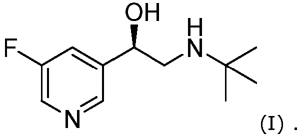

2. The salt according to Claim 1, wherein the tartrate comprises (2R,3R)-tartrate.

3. The salt according to Claim 2, wherein the tartrate essentially consists of (2R,3R)-tartrate.

4. The salt according to any one of the previous claims, wherein the salt has a purity of greater than 90%.

5. The salt according to any one of the previous claims, wherein the salt has a melting point of from 209 to 213 °C at atmospheric pressure.

6. The salt according to any one of the preceding claims for use in medicine.

7. A pharmaceutical composition comprising the salt as defined in any one of the preceding claims, and optionally one or more pharmaceutically acceptable adjuvant, diluent and/or carrier.

8. The pharmaceutical composition according to Claim 7, wherein the compound of formula I has an enantiomeric excess of at least 90%.

9. A salt as defined any one of Claims 1 to 5 for use in the treatment of hyperglycaemia or a disorder **characterized by** hyperglycaemia.

10. A pharmaceutical composition as defined in any one of Claims 7 to 8 for use in the treatment of hyperglycaemia or a disorder **characterized by** hyperglycaemia.

11. The salt or composition for use according to any one of Claims 9 and 10, wherein the hyperglycaemia or disorder **characterised by** hyperglycaemia is, or is **characterised by**, the patient displaying severe insulin resistance.

12. The salt or composition for use according to any one of Claims 9 and 10, wherein the disorder **characterised by** hyperglycaemia is selected from the group consisting of Type 2 diabetes, Rabson-Mendenhall syndrome, Donohue's syndrome (leprechaunism), Type A and Type B syndromes of insulin resistance, the HAIR-AN (hyperandrogenism, insulin resistance, and acanthosis nigricans) syndromes, pseudoacromegaly, and lipodystrophy.

13. A combination product comprising:
(a) a salt as defined in any one of Claims 1 to 5; and
(b) one or more other therapeutic agent that is useful in the treatment of hyperglycaemia or a disorder **characterised by** hyperglycaemia,
wherein each of components (a) and (b) is formulated in admixture, optionally with one or more pharmaceutically-acceptable adjuvant, diluent or carrier.

14. A kit-of-parts comprising:
(a) a pharmaceutical composition as defined in any one of Claims 7 to 8, and
(b) one or more other therapeutic agent that is useful in the treatment of hyperglycaemia or a disorder **characterised by** hyperglycaemia, optionally in admixture with one or more pharmaceutically-acceptable adjuvant, diluent or carrier,
which components (a) and (b) are each provided in a form that is suitable for administration in conjunction with the other.

15. A process for the preparation of the salt as defined in any one of Claims 1 to 5, comprising the step of reacting a compound of formula (I): 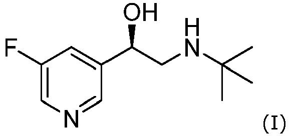 with tartaric acid or a solution of tartaric acid, such as wherein the solvent of the solution of tartaric acid is ethanol or an ethanol-water mixture.

## Patentansprüche

1. Hemi-Tartrat-Salz einer Verbindung der Formel I:

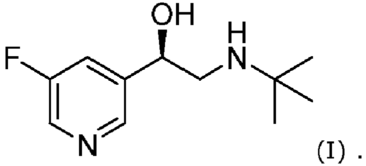

2. Salz nach Anspruch 1, wobei das Tartrat (2*R*, 3*R*)-Tartrat umfasst.

3. Salz nach Anspruch 2, wobei das Tartrat im Wesentlichen aus (2*R*, 3*R*)-Tartrat besteht.

4. Salz nach einem der vorstehenden Ansprüche, wobei das Salz eine Reinheit von mehr als 90 % aufweist.

5. Salz nach einem der vorstehenden Ansprüche, wobei das Salz einen Schmelzpunkt von 209 bis 213 °C bei atmosphärischem Druck aufweist.

6. Salz nach einem der vorstehenden Ansprüche zur Verwendung in der Medizin.

7. Pharmazeutische Zusammensetzung, umfassend das Salz, wie in einem der vorstehenden Ansprüche definiert, und wahlweise einen oder mehrere pharmazeutisch verträgliche Hilfsstoffe, ein oder mehrere pharmazeutisch verträgliche Verdünnungsmittel und/oder einen oder mehrere pharmazeutisch verträgliche Träger.

8. Pharmazeutische Zusammensetzung nach Anspruch 7, wobei die Verbindung der Formel I einen enantiomeren Überschuss von mindestens 90 % aufweist.

9. Salz nach einem der Ansprüche 1 bis 5 zur Verwendung bei der Behandlung von Hyperglykämie oder einer Störung, **gekennzeichnet durch** Hyperglykämie.

10. Pharmazeutische Zusammensetzung, wie in einem der Ansprüche 7 bis 8 definiert, zur Verwendung bei der Behandlung von Hyperglykämie oder einer Störung, **gekennzeichnet durch** Hyperglykämie.

11. Salz oder Zusammensetzung zur Verwendung nach einem der Ansprüche 9 und 10, wobei die Hyperglykämie oder Störung,
**gekennzeichnet durch** Hyperglykämie, darin besteht oder
**dadurch gekennzeichnet ist**, dass der Patient eine schwere Insulinresistenz aufweist.

12. Salz oder Zusammensetzung zur Verwendung nach einem der Ansprüche 9 und 10, wobei die Störung, **gekennzeichnet durch** Hyperglykämie, ausgewählt ist aus der Gruppe bestehend aus Typ-2-Diabetes, Rabson-Mendenhall-Syndrom, Donohue-Syndrom (Leprechaunismus), Insulinresistenz-Syndrom Typ A und Typ B, den HAIR-AN-Syndromen (Hyperandrogenismus, Insulinresistenz und Acanthosis nigricans), Pseudoakromegalie und Lipodystrophie.

13. Kombinationsprodukt, umfassend:
(a) ein Salz nach einem der Ansprüche 1 bis 5; und
(b) ein oder mehrere andere therapeutische Mittel, die bei der Behandlung von Hyperglykämie oder einer Störung, **gekennzeichnet durch** Hyperglykämie, nützlich sind, wobei jede der Komponenten (a) and (b) in Beimischung formuliert ist, wahlweise mit einem oder mehreren pharmazeutisch verträglichen Hilfsstoffen, Verdünnungsmitteln oder Trägern.

14. Teilesatz, umfassend:
(a) eine pharmazeutische Zusammensetzung nach einem der Ansprüche 7 bis 8, und
(b) ein oder mehrere andere therapeutische Mittel, die bei der Behandlung von Hyperglykämie oder einer Störung, **gekennzeichnet durch** Hyperglykämie, nützlich sind, wahlweise in Beimischung mit einem oder mehreren pharmazeutisch verträglichen Hilfsstoffen, Verdünnungsmitteln oder Trägern, wobei die Komponenten (a) und (b) jeweils in einer Form bereitgestellt sind, die für die Verabreichung in Verbindung mit der anderen geeignet ist.

15. Verfahren zur Herstellung des Salzes nach einem der Ansprüche 1 bis 5, umfassend den Schritt des Umsetzens einer Verbindung der Formel (I): 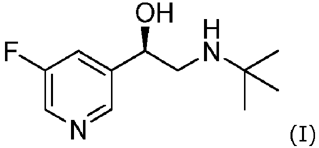 mit Weinsäure oder einer Lösung von Weinsäure, wie wobei das Lösungsmittel der Lösung von Weinsäure Ethanol oder ein Ethanol-WasserGemisch ist.

## Revendications

1. Sel hémi-tartrate d'un composé de formule I :

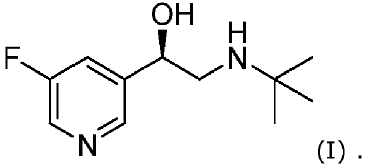

2. Sel selon la revendication 1, dans lequel le tartrate comprend (2*R*, 3*R*)-tartrate.

3. Sel selon la revendication 2, dans lequel le tartrate consiste essentiellement du (2*R*, 3*R*)-tartrate.

4. Sel selon l'une quelconque des revendications précédentes, dans lequel le sel a une pureté supérieure à 90 %.

5. Sel selon l'une quelconque des revendications précédentes, dans lequel le sel a un point de fusion de 209 à 213 °C à pression atmosphérique.

6. Sel selon l'une quelconque des revendications précédentes pour utilisation en médecine.

7. Composition pharmaceutique comprenant le sel tel que défini selon l'une quelconque des revendications précédentes, et éventuellement un ou plusieurs adjuvants, diluants et/ou supports pharmaceutiquement acceptables.

8. Composition pharmaceutique selon la revendication 7, dans laquelle le composé de formule I présente un excès énantiomérique d'au moins 90 %.

9. Sel tel que défini selon l'une quelconque des revendications 1 à 5 pour une utilisation dans le traitement de l'hyperglycémie ou d'un trouble **caractérisé par** l'hyperglycémie.

10. Composition pharmaceutique telle que définie selon l'une quelconque des revendications 7 à 8 pour utilisation dans le traitement de l'hyperglycémie ou d'un trouble **caractérisé par** l'hyperglycémie.

11. Sel ou composition pour utilisation selon l'une quelconque des revendications 9 et 10, dans laquelle l'hyperglycémie ou le trouble **caractérisé par** une hyperglycémie est, ou est **caractérisé par, le fait que** le patient présente une résistance à l'insuline sévère.

12. Sel ou composition pour utilisation selon l'une quelconque des revendications 9 et 10, dans laquelle le trouble **caractérisé par** une hyperglycémie est choisi dans le groupe constitué du diabète de type 2, du syndrome de Rabson-Mendenhall, du syndrome de Donohue (lépréchaunisme), des syndromes de résistance à l'insuline de type A et de type B, des syndromes HAIR.-AN (hyperandrogénie, résistance à l'insuline et acanthosis nigricans), de pseudo-acromégalie et de lipodystrophie.

13. Produit de combinaison comprenant :
(a) un composé tel que défini selon l'une quelconque des revendications 1 à 5 ; et
(b) un ou plusieurs autres agents thérapeutiques qui sont utiles dans le traitement de l'hyperglycémie ou d'un trouble **caractérisé par** l'hyperglycémie, dans lequel chacun des composants (a) et (b) est formulé en mélange, éventuellement avec un ou plusieurs adjuvants, diluants ou supports pharmaceutiquement acceptables.

14. Kit de composants comprenant :
(a) une composition pharmaceutique telle que définie selon l'une quelconque des revendications 7 à 8, et
(b) un ou plusieurs autres agents thérapeutiques utiles dans le traitement de l'hyperglycémie ou d'un trouble **caractérisé par** une hyperglycémie, éventuellement en mélange avec un ou plusieurs adjuvants, diluants ou véhicules pharmaceutiquement acceptables, lesquels composants (a) et (b) sont chacun fournis sous une forme qui est appropriée pour une administration conjointement avec l'autre.

15. Procédé de préparation du sel tel que défini selon l'une quelconque des revendications 1 à 5, comprenant l'étape de réaction d'un composé de formule (I) : 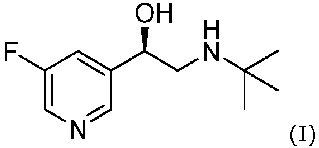 avec de l'acide tartrique ou une solution d'acide tartrique, tel que dans lequel le solvant de la solution d'acide tartrique est l'éthanol ou un mélange éthanol-eau.
